(19) Europäisches Patentamt
European Patent Office
Office européen des brevets

(11) **EP 2 568 274 B1**

(12) **EUROPEAN PATENT SPECIFICATION**

(45) Date of publication and mention
of the grant of the patent:
**23.10.2019 Bulletin 2019/43**

(51) Int Cl.:
***G01N 21/17*** *(2006.01)* ***A61B 5/00*** *(2006.01)*

(21) Application number: **12005925.8**

(22) Date of filing: **16.08.2012**

(54) **Use of a photoacoustic contacting material and method of performing photoacoustic tomography**

Verwendung eines photoakustischen Kontaktmaterials und Verfahren zur Durchführung einer photoakustischen Tomographie

Utilisation d'un matériau de contact photo-acoustique et procédé de tomographie photo-acoustique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2011 JP 2011197254**

(43) Date of publication of application:
**13.03.2013 Bulletin 2013/11**

(73) Proprietor: **Canon Kabushiki Kaisha**
**Ohta-ku**
**Tokyo (JP)**

(72) Inventor: **Wada, Yoshiko**
**Tokyo (JP)**

(74) Representative: **WESER & Kollegen**
**Patentanwälte PartmbB**
**Radeckestraße 43**
**81245 München (DE)**

(56) References cited:
**WO-A1-2011/055501    WO-A2-2009/083846**
**JP-A- 2007 319 576    JP-A- 2010 125 260**
**US-A- 5 312 484    US-A1- 2004 082 070**
**US-B2- 6 574 490**

• **JEREMY C HEBDEN ET AL: "A soft deformable tissue-equivalent phantom for diffuse optical tomography; A soft phantom for diffuse optical tomography", PHYSICS IN MEDICINE AND BIOLOGY, INSTITUTE OF PHYSICS PUBLISHING, BRISTOL GB, vol. 51, no. 21, 7 November 2006 (2006-11-07), pages 5581-5590, XP020096019, ISSN: 0031-9155, DOI: 10.1088/0031-9155/51/21/013**

**Description**

BACKGROUND OF THE INVENTION

Field of the Invention

One disclosed aspect of the embodiments relates to a photoacoustic contacting material, subsequently also designated as "photoacoustic matching material", used in medical diagnostics to optically and acoustically contact a photoacoustic tomography apparatus and the like to a surface of a subject to be examined.

Description of the Related Art

A photoacoustic tomography (PAT) apparatus used for medical diagnosis has been offered as an apparatus for imaging an inside of an object to be examined (test object) by using ultrasonic wave (acoustic wave). The photoacoustic tomography apparatus irradiates the test object with laser pulse light, receives photoacoustic waves resulted from absorption of energy of radiated light in tissue of the test object with a receiving element (electromechanical conversion element such as piezoelement) provided in a probe, and makes an image of information that is relevant to optical property values in the inside of the test object.

Japanese Patent Application Laid-Open No. 2010-075681 discusses a constitution of a photoacoustic tomography apparatus in which a photoacoustic matching material is provided between a receiving element and a test object.

A matching of a photoacoustic impedance between the test object and the probe is formed by providing the photoacoustic matching material between the test object and the receiving element in technology described in Japanese Patent Application Laid-Open No. 2010-075681, but further improvement of the photoacoustic matching material is required. Specifically, it is required for the photoacoustic matching material that not only the photoacoustic impedance is considered but also optical properties such as reflection and transmission for the light irradiating the test object are considered. Further, good tactual sensation for the test object is also desired for the photoacoustic matching material because of being often used in direct contact with the test object such as a human body.

SUMMARY OF THE INVENTION

A photoacoustic contacting material used in one embodiment includes water, a thickener, and a light scattering member. The photoacoustic contacting material used in an embodiment has water as a major component among the water, the thickener, and the light scattering member.

The present invention in its first aspect provides, as specified in claims 1 to 13, a use of a photoacoustic contacting material in medical diagnostics to optically and acoustically contact a photoacoustic tomography apparatus to a surface of a subject to be examined, and further provides, in its second aspect, a method of performing photoacoustic tomography as specified in claim 14.

According to an embodiment, it is possible that the therein used photoacoustic matching material not only has a good acoustic impedance property but also has good optical properties for the light radiating to the test object. Further, it is possible that the photoacoustic matching material has a good tactual sensation for the test object.

Further features and aspects of the disclosure will become apparent from the following detailed description of exemplary embodiments with reference to the attached drawings.

BRIEF DESCRIPTION OF THE DRAWINGS

The accompanying drawings, which are incorporated in and constitute a part of the specification, illustrate exemplary embodiments, features, and aspects of the disclosure and, together with the description, serve to explain the principles of the disclosure.

Fig. 1 is a view illustrating a photoacoustic tomography apparatus with use of a photoacoustic contacting material according to the invention.

Fig. 2 is a partially magnified view illustrating the photoacoustic tomography apparatus with use of a photoacoustic contacting material according to the invention.

DESCRIPTION OF THE EMBODIMENTS

Various exemplary embodiments, features, and aspects of the disclosure will be described in detail below with reference to the drawings.

A photoacoustic matching material used in an embodiment will be described below with reference to the drawings, and first a photoacoustic tomography apparatus in which this photoacoustic matching material is used will be described.

The photoacoustic tomography apparatus used in the present embodiment is an apparatus utilizing a photoacoustic effect that an acoustic wave generated by irradiating a test object with light (electromagnetic wave) is received and the information of the test object is acquired as image data. The acoustic wave is typically the ultrasonic wave and includes those elastic waves referred to as sound waves, ultrasonic waves, photoacoustic waves, and photo-ultrasonic waves.

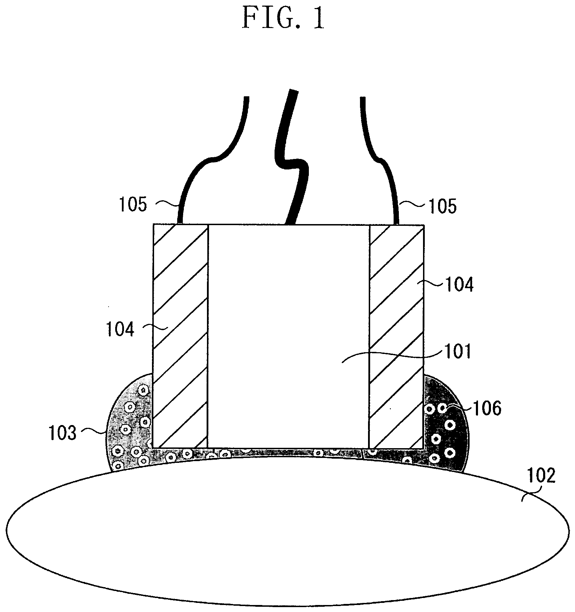

In Fig. 1, the photoacoustic tomography apparatus includes a light source not shown in the figure, a bundle fiber 105, a light irradiation unit 104, a photoacoustic matching material 103, a probe 101, a signal processing unit not shown in the figure, and a display unit not shown in the figure. The photoacoustic matching material includes a light scattering body 106 that is dispersed irregularly in the material and is a light scattering member, and is applied onto a surface of the test object. The probe 101 receives the acoustic wave from the test object 102 through the acoustic matching material 103.

The photoacoustic tomography apparatus generates pulse light from the light source not shown in the figure, and irradiates the test object 102 with the pulse light from the light irradiation unit 104 through the bundle fiber 105 and the photoacoustic matching material 103. An absorber in the test object 102 generates the photoacoustic wave by the pulse light radiated into the test object 102. The photoacoustic wave passes through the photoacoustic matching material 103 and is received by the probe 101.

The probe 101 and the subsequent signal processing unit convert a photoacoustic signal to an electric signal, amplify the signal, and perform delay processing and rearrangement to prepare an absorption coefficient distribution in the test object 102. For example, back projection in a time domain or Fourier domain typically used for tomography technology may be utilized as a method of rearrangement. Subsequently, the display unit displays the prepared absorption coefficient distribution.

The light irradiation unit 104 radiates the pulse light that is output from the light source and passes through the bundle fiber 105. The light to be radiated is near-infrared light with wavelength of about 750 to 1100 nm. The light emitted from the light irradiation unit 104 enters the test object 102 through the photoacoustic matching material 103. The light irradiation unit 104 comes into contact with a side face of the probe 101 in Fig. 1, but the light irradiation unit 104 may be in no contact with the probe 101, and they may be arranged with some angle.

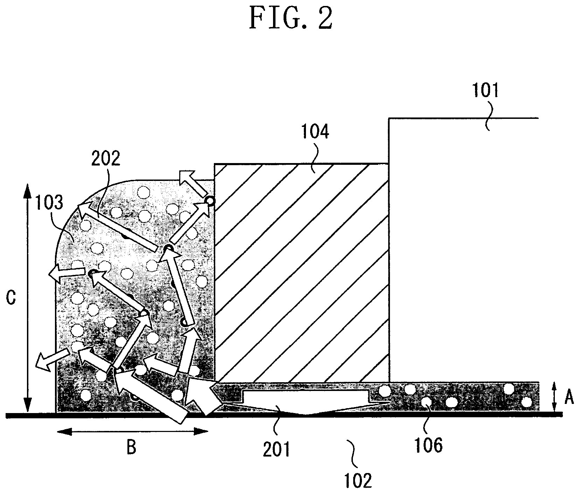

The photoacoustic matching material 103 is applied onto the surface of the test object. The light irradiation unit 104 irradiates the test object 102 with the light and the probe 101 acquires the acoustic wave from the test object 102, through the photoacoustic matching material 103. A thickness of the photoacoustic matching material 103 between the light irradiation unit 104 and the test object is typically about 0.5 mm or less because the light irradiation unit 104 often emits the light with being pressed to the test object 102. The light irradiation unit 104 is pressed after the photoacoustic matching material is applied onto the surface of the test object, and the photoacoustic matching material 103 around the light irradiation unit is applied in a size of about 10 mm from the periphery of the light irradiation unit. A positional relation of the light irradiation unit 104, the photoacoustic matching material 103, and the test object 102 is shown in Fig. 2. The size of this photoacoustic matching material corresponds to a distance B in Fig. 2.

The photoacoustic matching material in the present embodiment includes water, a thickener, and a light scattering member. Thus, the light 201 entered from the light irradiation unit 104 partially exits outside from the photoacoustic matching material 103, but the light is scattered by the light scattering body 106 that is the light scattering member in the photoacoustic matching material 103, and thus its energy density is reduced. This further enhances the safety for an operator and the subject. This will be described in detail.

The photoacoustic tomography apparatus generally uses laser as the light source for irradiating the test object with the light. JIS C6802 is provided as a rule for handling the device using the laser. In this rule, maximum permissible exposure (MPE) for retina and MPE for skin are defined as permissible safe levels when the eye and the skin are irradiated with the laser light. The laser having the energy density equal to or less than MPE for the skin is necessary to be used in the laser device used for the photoacoustic tomography apparatus.

However, the MPE for the retina is much smaller than the MPE for the skin. Thus, even when the test object is irradiated with the light at a level equal to or less than the MPE for the test object, i.e., the skin in order to acquire the photoacoustic wave, the energy density of the light leaked from the photoacoustic matching material potentially exceeds the MPE for the retina. However, in the photoacoustic matching material in the present embodiment, the energy density of the light is sufficiently reduced by the light scattering body 106, i.e., the photoacoustic matching material itself may reduce the energy density of the light. Thus, the safety for the operator and the subject may further be enhanced without giving an additional contrivance to the photoacoustic tomography apparatus.

Further the photoacoustic matching material in the present embodiment includes the water as the major component among the water, the thickener, and the light scattering member. This enables the photoacoustic matching material to be used without unpleasant sensation in terms of tactile sensation and odor even if the photoacoustic matching material is come into direct contact with the test object, particularly a human body.

The photoacoustic matching material 103 is further described below. Carboxyvinyl polymers, carboxymethyl-cellulose, acrylate methyl ester copolymers, xanthan gum, and the like may be used as the thickener of the photoacoustic matching material 103. The photoacoustic matching material 103 may contain compositions such as moisture-holding agents, preservatives, and pH adjusters in addition to the water, the thickener, and the light scattering body 106 that is the light scattering member. For example, glycerine, para-hydroxybenzoate ester, and sodium hydroxide may be contained. A particle diameter of the light scattering body 106 to be added may be approximately equivalent to or less than a wavelength of the light to be radiated, and may be 1 nm or more and 3 $\mu$m or less in average.

Inorganic fine particles and organic fine particles may be used for the light scattering body 106.

Examples of the inorganic fine particle include inorganic particles such as silica, diatomaceous earth, alumina, zinc oxide, titania (titanium oxide), zirconia, calcium oxide, magnesium oxide, gold, silver.

Examples of the organic fine particle include publicly known organic resin fine particles such as vinyl resins, urethane resins, epoxy resins, ester resins, polyamide, polyimide, silicone resins, fluorine resins, phenol resins, melamine resins, benzoguanamine-based resins, urea resins, aniline resins, ionomer resins, polycarbonate, cellulose, and mixtures thereof. Inorganic materials and organic materials may be used without limiting to either ones alone and may be combined in the light scattering body 106 to be added to the photoacoustic matching material 103.

When the photoacoustic matching material 103 has an absorbance for the radiated wavelength or a light absorber is added to the photoacoustic matching material 103, the entered light exits with scattering and with the light intensity attenuating.

The light absorber to be added to the photoacoustic matching material 103 includes pigments and dyes such as methylene blue and indocyanine green (ICG). The light absorber and the light scattering body may be the same substance, which includes carbon black, graphite, carbon pigments, and mixed atomic valence metal complexes that absorb the near-infrared light.

A degree of the scattering of the light that exits from the photoacoustic matching material 103 is determined by a distance where the light is propagated in the photoacoustic matching material 103 and a light-scattering coefficient. The light-scattering coefficient varies depending on a size, a refractive index, and a concentration of the light scattering body.

When a thickness (A in Fig. 2) of the photoacoustic matching material 103 between the light irradiation unit 104 and the test object is about 0.5 mm, a scattering coefficient of the light which passes through the distance to perform isotropic scattering is 2/mm.

When the photoacoustic matching material 103 around the light irradiation unit 104 has a size of about 10 mm (B in Fig. 2) and a thickness of about 1 mm (C in Fig. 2), the light incident from the light irradiation unit 104 to exit from the photoacoustic matching material 103 passes through a distance of about 1 mm to 10 mm. The scattering coefficient, when the isotropic scattering is performed on the light that passes through 10 mm, is 0.1/mm. Further the scattering coefficient, when the isotropic scattering is performed on the light that passes through 1 mm, is 1/mm. Generally, it is useful that the light (light that passes the distance A in Fig. 2) emitted from the photoacoustic matching material 103 toward the test object 102 is straight ahead light, and it is useful that the isotropic scattering is sufficiently performed on the light (the light that passes the distances B and C in Fig. 2)emitted towards directions other than the test object 102 . Thus, the scatter coefficient may be advantageously 0.1/mm or more and 2.0/mm or less, and more advantageously 1.0/mm or more and 2.0/mm or less.

When the photoacoustic matching material 103 has an absorption coefficient, the light emitted from the photoacoustic matching material 103 is attenuated in relation to a distance where the light migrates in the photoacoustic matching material 103 and a size of the absorption coefficient. The larger the scattering coefficient is, the distance where the light migrates in the photoacoustic matching material 103 is more increased. The attenuation of the light from the photoacoustic matching material 103 is represented by a value of an damping coefficient calculated from the light scattering coefficient and the light absorption coefficient (equation 1).

$$\mu_{\mathrm{eff}} = \sqrt{3 \cdot \mu_{\mathrm{a}} \cdot \left( \mu_{\mathrm{a}} + \mu_{\mathrm{s}}' \right)} \qquad (1)$$

wherein, the damping coefficient, the absorption coefficient, and the scattering coefficient are represented by $\mu$eff, $\mu$a, and $\mu$s', respectively.

Here, it is advantageous that the light emitted from the photoacoustic matching material 103 toward the test object 102 is not so attenuated whereas the light emitted toward the direction other than the test object 102 is strongly attenuated down to approximately MPE for the retina. This is because the difference between the MPE for the retina and the MPE for the skin ranges from several to several hundred thousand times.

In the photoacoustic tomography apparatus, the test object 102 is irradiated with the light based on the MPE for the skin because it is desirable to obtain the large signal from the test object 102. However, it is desirable that the light that exits from the photoacoustic matching material 103 is weakened down to approximately MPE for the retina of the operator and the test object.

When a light source having a wavelength of 797 nm, a pulse width of 10 ns, and a light emission frequency of 10 Hz is used, the MPE for the skin is 313 $(J/m^2)$. However, the MPE for the retina is not determined uniquely. The reason for it is that the MPE for the retina varies depending on a range of a diffused light source and a distance to the subject. The MPE for the retina becomes high when the diffused light source is small and the operator and the subject are away from the diffused light source.

For example, upon using the photoacoustic tomography apparatus, when the operator and the subject are about 1 m away from the diffused light source and observe the diffused light source with a width of 5 mm from a direction of 60 degrees, the MPE is 0.14 $(J/m^2)$, and when the diffused light source with a width of 10 mm is observed from a direction of 45 degrees, the MPE is 0.39 $(J/m^2)$. Even when the subject gets close or the size of the diffused light source is increased, an upper limit of the MPE is set to be 2.8 $(J/m^2)$. As described above, the MPE for the retina is 0.14 $(J/m^2)$ to 2.8 $(J/m^2)$ in the photoacoustic tomography apparatus under a circumstance generally employed. As described above, the MPE for the retina may be set in more detail depending on statuses of use of the photoacoustic tomography apparatus, e.g., the size of the diffused light source and the distance between the diffused light source and the subject. The advantageous range of the damping coefficient (0.47/mm to 0.77/mm) and the absorption coefficient (0.036/mm to 0.40/mm) of the photoacoustic matching material 103 are determined by the MPE for the retina.

It is desirable that the scattering coefficient, the absorption coefficient and the damping coefficient of the photoacoustic matching material 103 are appropriately set in suitable ranges depending on the wavelength used in the photoacoustic apparatus, the size of the diffused light source (surface range of photoacoustic matching material 103) for the subject, and a predicted value of the distance.

Exemplary embodiments will be described below, but the disclosure is not limited to these embodiments, and materials, compositions, reaction conditions, and the like may be changed freely as long as the photoacoustic matching material 103 having the similar function and effect is obtained.

A first exemplary embodiment will be described. A photoacoustic matching material and a photoacoustic tomography apparatus used in the present exemplary embodiment are shown in Fig. 1. A photoacoustic matching material 103 is used between a light irradiation unit 104 and a test object 102 in the photoacoustic tomography apparatus.

A laser pulse light source having a wavelength of 797 nm, a pulse width of 10 ns, and a light emission frequency of 10 Hz is used as a light source in the present exemplary embodiment. Ultrasonic gel (LOGICLEAN, GE Yokokawa Medical) is used as a base material of the photoacoustic matching material 103. The ultrasonic gel includes water and a thickener, and its major component is the water. Thus, it is odorless and non-sticky, and no unpleasant sensation is given even when the gel is used with coming into direct contact with the test object. Titanium oxide having a particle diameter of 0.21 μm in an amount of 0.3% by weight as the light scattering body 106 that is the light scattering member is added to and dispersed in the ultrasonic gel. As a result, the scattering coefficient, the absorption coefficient, and the damping coefficient are 1.0 (/mm), 0.0081 (/mm), and 0.16 (/mm), respectively. Also after adding the light scattering body 106 that is the light scattering member, no large change is found in odor and tactile sensation.

A case is supposed in which the operator and the subject observes the diffused light source (photoacoustic matching material) on the surface of the test object at a position 1 m apart from it with an angle of 45 degrees. The diffused light source is supposed to be the light leaked from between the light irradiation unit 104 and the test object (A in Fig. 2), and its size is supposed to be 0.5 mm. The photoacoustic matching material 103 has a size of 10 mm (B in Fig. 2), and is applied in a thickness of about 10 mm (C in Fig. 2)at the periphery of the light irradiation unit.

A case of using a photoacoustic matching material 103 without containing the light scattering body 106 and a case of using the photoacoustic matching material 103 as in the present exemplary embodiment are compared and examined.

Considering the absorption coefficient, the damping coefficient, and a light exit area in each case, the energy density of the light in the case of using the photoacoustic matching material 103 as in the first exemplary embodiment is about 0.007 times compared with the case of using the photoacoustic matching material without containing the light scattering body 106. Thus, by the use of the photoacoustic matching material 103 as in the first exemplary embodiment, an energy irradiation of the irradiated light is reduced from 313 (J/m2) to 2.3 (J/m2), and the safety for the operator and the subject may be further enhanced.

A second exemplary embodiment will be described. The photoacoustic matching material 103 obtained by adding titanium oxide that is the light scattering body 106 to the ultrasonic gel containing the water as the major component and further containing the thickener was described in the first exemplary embodiment. The photoacoustic matching material 103 obtained by adding not only the light scattering body 106 but also a light absorber that is light absorption member will be described in the present exemplary embodiment. The photoacoustic matching material 103 and the photoacoustic tomography apparatus using the same in the present exemplary embodiment are shown in Fig. 2. In the present embodiment, carbon black that is the light scattering body 106 as well as the absorber that is the light absorption member is added in an amount of 0.01% by weight to the photoacoustic matching material 103. As a result, the scattering coefficient, the damping coefficient, and the attenuation coefficient are 1.0 (/mm), 0.133 (/mm), and 0.67 (/mm), respectively. By adding the absorber, the energy density of the light that exited from the photoacoustic matching material 103 may be reduced compared with the first exemplary embodiment.

The damping coefficient of the photoacoustic matching material 103 is 0.16 (/mm) in the first exemplary embodiment and 0.133 (/mm) in the second exemplary embodiment. Thus, the energy density of the light radiated from the photoacoustic matching material 103 used in the present embodiment is 0.006 times compared with that in the first exemplary embodiment. Thus, whereas the MPE for the retina is 0.39 (J/m2), the energy density of the light radiated from the photoacoustic matching material 103 in the present exemplary embodiment is maximally 0.013 (J/m2), indicating that the safety for the operator and the subject may further be enhanced.

In the present exemplary embodiment, the light scattering member and the light absorption member are realized in the same member by using carbon black having both a light scattering function and a light absorption function, but the light scattering member and the light absorption member are not limited thereto, and may be composed of different members.

While the disclosure has been described with reference to exemplary embodiments, it is to be understood that the disclosure is not limited to the disclosed exemplary embodiments but defined by the following claims.

## Claims

1. Use of a photoacoustic contacting material (103) in medical diagnostics to optically and acoustically contact a photoacoustic tomography apparatus (101, 104, 105) to a surface of a subject (102) to be examined, the photoacoustic contacting material comprising water and a thickener;

   **characterized in that** the photoacoustic contacting material further comprises a light scattering member (106); and
   **in that** a light scattering coefficient of the photoacoustic contacting material is equal to or greater than 0.1/mm and equal to or smaller than 2.0/mm.

2. The use according to claim 1, wherein the photoacoustic contacting material includes the water as a major component.

3. The use according to claim 1, wherein the thickener is composed of a carboxyvinyl polymer, carboxymethyl-cellulose, an acrylate methyl ester copolymer, or xanthan gum.

4. The use according to claim 1, wherein an average particle diameter of the light scattering member is equal to or greater than 1 nm and equal to or smaller than 3 $\mu$m.

5. The use according to claim 1, wherein the light scattering member is composed of an inorganic material.

6. The use according to claim 5, wherein the inorganic material is composed of silica, diatomaceous earth, alumina, zinc oxide, titanium oxide, zirconia, calcium oxide, magnesium oxide, gold, or silver.

7. The use according to claim 5, wherein the inorganic material is titanium oxide.

8. The use according to claim 1, wherein the light scattering member is composed of an organic material.

9. The use according to claim 8, wherein the organic material is composed of vinyl resins, urethane resins, epoxy resins, ester resins, polyamide, polyimide, silicone resins, fluorine resins, phenol resins, melamine resins, benzoguanamine-based resins, urea resins, aniline resins, ionomer resins, polycarbonate, cellulose, or mixtures thereof.

10. The use according to claim 1, wherein the light scattering member is carbon black.

11. The use according to claim 1, wherein the photoacoustic contacting material includes a light absorption member.

12. The use according to claim 11, wherein the light absorption member is carbon black.

13. The use according to claim 1, wherein the photoacoustic contacting material further comprises a moisture-holding agent, a preservative, or a pH adjuster.

14. A method of performing photoacoustic tomography with a photoacoustic tomography apparatus (101, 104, 105) on an object (102);

  wherein the tomography apparatus contacts a surface of the object via a contacting material (103) which is the same material as the photoacoustic contacting material defined in any one of claims 1 to 13;
  irradiates (104) the object through the contacting material with pulsed light; and
  receives (101) from the subject through the contacting material a photoacoustic wave generated by said pulsed light in the subject.

**Patentansprüche**

1. Verwendung eines photoakustischen Kontaktmaterials (103) in medizinischer Diagnostik, um eine Vorrichtung zur photoakustischen Tomographie (101, 104, 105) mit einer zu untersuchenden Fläche eines Subjekts (102) optisch und akustisch in Kontakt zu bringen, wobei das photoakustische Kontaktmaterial Wasser und ein Verdickungsmittel umfasst:

  **gekennzeichnet dadurch, dass** das photoakustische Kontaktmaterial außerdem ein Lichtstreuungselement (106) umfasst; und
  dass ein Lichtstreuungskoeffizient des photoakustischen Kontaktmaterials gleich oder größer als 0,1/mm und gleich oder kleiner als 2,0/mm ist.

2. Verwendung nach Anspruch 1, wobei das photoakustische Kontaktmaterial das Wasser als eine Hauptkomponente enthält.

3. Verwendung nach Anspruch 1, wobei das Verdickungsmittel aus einem Carboxyvinylpolymer, Carboxymethyl-Cellulose, einem Acrylat-MethylEster-Copolymer oder Xanthangummi zusammengesetzt ist.

4. Verwendung nach Anspruch 1, wobei ein mittlerer Teilchendurchmesser des Lichtstreuungselements gleich oder größer als 1 nm und gleich oder kleiner als 3 $\mu$m ist.

5. Verwendung nach Anspruch 1, wobei das Lichtstreuungselement aus einem anorganischen Material zusammengesetzt ist.

6. Verwendung nach Anspruch 5, wobei das anorganische Material aus Siliciumoxid, Diatomeenerde, Aluminiumoxid, Zinkoxid, Titanoxid, Zirconiumoxid, Calziumoxid, Magnesiumoxid, Gold oder Silber zusammengesetzt ist.

7. Verwendung nach Anspruch 5, wobei das anorganische Material Titanoxid ist.

8. Verwendung nach Anspruch 1, wobei das Lichtstreuungselement aus organischem Material zusammengesetzt ist.

9. Verwendung nach Anspruch 8, wobei das organische Material aus Vinylharzen, Urethanharzen, Epoxyharzen, Esterharzen, Polyamid, Polyimid, Silikonharzen, Fluorharzen, Phenolharzen, Melaminharzen, Benzoguanamin-basierenden Harzen, Harnstoffharzen, Anilinharzen, Ionomerharzen, Polycarbonat, Cellulose oder Mischungen daraus zusammengesetzt ist.

10. Verwendung nach Anspruch 1, wobei das Lichtstreuungselement Ruß ist.

11. Verwendung nach Anspruch 1, wobei das photoakustische Kontaktmaterial ein Lichtabsorptionselement enthält.

12. Verwendung nach Anspruch 11, wobei das Lichtabsorptionselement Ruß ist.

13. Verwendung nach Anspruch 1, wobei das photoakustische Kontaktmaterial ferner ein Feuchtigkeitshaltemittel, ein Konservierungsmittel oder ein Mittel zur Einstellung eines pH-Wertes umfasst.

14. Verfahren zum Durchführen einer photoakustischen Tomographie mit einer Vorrichtung zur photoakustischen Tomographie (101, 104, 105) auf einem Objekt (102);

wobei die Vorrichtung zur Tomographie eine Fläche des Objekts über ein Kontaktmaterial (103) kontaktiert, das dasselbe Material wie das photoakustische Kontaktmaterial nach einem der Ansprüche 1 bis 13 ist; das Objekt durch das Kontaktmaterial mit gepulstem Licht bestrahlt (104); und vom Subjekt durch das Kontaktmaterial eine durch das gepulste Licht im Subjekt erzeugte photoakustische Welle empfängt (101).

**Revendications**

1. Utilisation d'un matériau de contact photo-acoustique (103) lors de diagnostics médicaux pour établir un contact optique et acoustique entre un appareil de tomographie photo-acoustique (101, 104, 105) et une surface d'un sujet (102) à examiner, le matériau de contact photo-acoustique comprenant de l'eau et un épaississant ;
**caractérisée en ce que** le matériau de contact photo-acoustique comprend en outre un élément de diffusion de lumière (106) ; et
**en ce qu'**un coefficient de diffusion de lumière du matériau de contact photo-acoustique est égal ou supérieur à 0,1/mm et égal ou inférieur à 2,0/mm.

2. Utilisation selon la revendication 1, dans laquelle le matériau de contact photo-acoustique comprend de l'eau en tant que constituant principal.

3. Utilisation selon la revendication 1, dans laquelle l'épaississant est constitué d'un polymère carboxyvinylique, d'une carboxyméthylcellulose, d'un copolymère d'ester méthylique d'acrylate, ou d'une gomme de xanthane.

4. Utilisation selon la revendication 1, dans laquelle un diamètre de particule moyen de l'élément de diffusion de lumière est égal ou supérieur à 1 nm et égal ou inférieur à 3 $\mu$m.

5. Utilisation selon la revendication 1, dans laquelle l'élément de diffusion de lumière est constitué d'un matériau inorganique.

6. Utilisation selon la revendication 5, dans laquelle le matériau inorganique est constitué de silice, de terre de diatomées, d'alumine, d'oxyde de zinc, d'oxyde de titane, de zircone, d'oxyde de calcium, d'oxyde de magnésium, d'or, ou d'argent.

7. Utilisation selon la revendication 5, dans laquelle le matériau inorganique est de l'oxyde de titane.

8. Utilisation selon la revendication 1, dans laquelle l'élément de diffusion de lumière est constitué d'un matériau organique.

9. Utilisation selon la revendication 8, dans laquelle le matériau organique est constitué de résines vinyliques, de résines uréthanes, de résines époxy, de résines d'esters, de polyamide, de polyimide, de résines de silicone, de résines fluorées, de résines phénoliques, de résines de mélamine, de résines à base de benzoguanamine, de résines d'urée, de résine d'aniline, de résines ionomères, de polycarbonate, de cellulose, ou de leurs mélanges.

10. Utilisation selon la revendication 1, dans laquelle l'élément de diffusion de lumière est du noir de carbone.

11. Utilisation selon la revendication 1, dans laquelle le matériau de contact photo-acoustique comprend un élément d'absorption de lumière.

12. Utilisation selon la revendication 11, dans laquelle l'élément d'absorption de lumière est du noir de carbone.

13. Utilisation selon la revendication 1, dans laquelle le matériau de contact photo-acoustique comprend en outre un agent de rétention d'eau, un conservateur, ou un agent d'ajustement du pH.

14. Procédé d'exécution d'une tomographie photo-acoustique au moyen d'un appareil de tomographie photo-acoustique (101, 104, 105) placé sur un objet (102) ;
dans lequel l'appareil de tomographie contacte une surface de l'objet par le biais d'un matériau de contact (103) qui est le même matériau que le matériau de contact photo-acoustique selon l'une quelconque des revendications 1 à 13 ;

expose (104) l'objet par l'intermédiaire du matériau de contact à un rayonnement de lumière puisée ; et

reçoit (101) du sujet, par l'intermédiaire du matériau de contact, une onde photo-acoustique générée par ladite lumière pulsée dans le sujet.

FIG. 1

FIG. 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010075681 A